(19) Europäisches Patentamt
European Patent Office
Office européen des brevets

(11) Publication number: 0 013 112
A1

(12) # EUROPEAN PATENT APPLICATION

(21) Application number: 79302920.8

(22) Date of filing: 17.12.79

(51) Int. Cl.³: C 07 C 119/042
C 08 G 18/70, C 08 L 97/02

(30) Priority: 27.12.78 GB 4997778

(43) Date of publication of application:
09.07.80 Bulletin 80/14

(84) Designated Contracting States:
BE DE FR GB IT SE

(71) Applicant: IMPERIAL CHEMICAL INDUSTRIES LIMITED
Imperial Chemical House Millbank
London SW1P 3JF(GB)

(72) Inventor: Redman, Richard Paul
75 Hilltop Avenue
Cheadle Hulme Cheshire(GB)

(74) Representative: Ricks, Michael James et al,
Imperial Chemical Industries Limited Legal Department:
Patents Thames House North Millbank
London SW1P 4QG(GB)

(54) **Emulsifiable compositions and aqueous emulsions of organic isocyanates, and process using them as binders for manufacturing lignocellulose sheets.**

(57) An aqueous emulsion comprising an organic isocyanate and a surfactant which is the reaction product of an organic di- or polyisocyanate with
(1) an alkoxy polyethylene glycol of formula $RO(CH_2CH_2O)_nH$ wherein R is an alkyl group of 1 to 20 carbon atoms, and n is at least 5 and, optionally,
(2) an isocyanate-reactive compound having one or more hydroxy, amino or carboxylic acid groups,
wherein the proportions of the said isocyanate-reactive compound (2) are from 0 to 50 parts by weight of isocyanate-reactive compound per 100 parts by weight of akoxy polyethylene glycol (1), and wherein the isocyanate function of the organic di- or polyisocyanate is completely reacted with the alkoxy polyethylene glycol and the isocyanate-reactive compound (if used).

The emulsions are useful as adhesives, surface coatings and especially as binders in the manufacture of chipboard.

EP 0 013 112 A1

Organic isocyanates are well known as binders, adhesives and surface coatings, for example in the manufacture of chipboard. Organic isocyanates used for such purposes are generally too viscous to be readily applied as such and it has been found to be very effective to apply the organic isocyanate in the form of an aqueous emulsion. Aqueous emulsions of organic isocyanates have previously been prepared which are stable in the sense that they do not readily separate into discrete layers of their main components. Such emulsions, however, are difficult to use for some applications as a result of degradation, for example by gelling by reaction of the water and the isocyanate to form a polyurea. This leads to a reduction in the time during which the emulsions remain workable, such time being referred to as the "pot life". Stable emulsions of adequate "pot life" are described for example in UK Patent Specification No.1444933 which discloses aqueous emulsions containing a surface active agent of formula $RO(CH_2CH_2O)_nCONHX$ wherein R is an alkyl group of from 1 to 4 carbon atoms, n is an integer such that the compound contains an average of at least 5 oxyethylene groups and X is the residue of a di- or poly-isocyanate and contains at least one free isocyanate group.

Surface active agents having free isocyanate groups cannot be incorporated in the aqueous phase prior to emulsification, and indeed it has hitherto proved difficult to prepare stable isocyanate emulsions of adequate "pot life" when the surfactant is incorporated into the aqueous phase. In particular there is a need for a surface active agent which is capable of being added to either the aqueous or the organic phase. This versatility is also desirably accompanied by the ability to formulate a family of such

surface active agents whose emulsion-forming properties may be varied to suit specific emulsion formulations.

According to the present invention there is provided an aqueous emulsion comprising an organic isocyanate and a surfactant which is the reaction product of an organic di- or polyisocyanate with
(1) an alkoxy polyethylene glycol of formula $RO(CH_2CH_2O)_nH$ wherein R is an alkyl group of 1 to 20 carbon atoms, and n is at least 5 and, optionally,
(2) an isocyanate-reactive compound having one or more hydroxy, amino or carboxylic acid groups,
wherein the proportions of the said isocyanate-reactive compound (2) are from 0 to 50 parts by weight of isocyanate-reactive compound per 100 parts by weight of alkoxy polyethylene glycol (1), and wherein the isocyanate function of the organic di- or polyisocyanate is completely reacted with the alkoxy polyethylene glycol and the isocyanate-reactive compound (if used).

The organic isocyanate which constitutes the aqueous emulsion and the organic di- or polyisocyanate which is reacted with the alkoxy polyethylene glycol and the isocyanate-reactive compound (if used) to form the surface active agent may be the same or different.

The alkyl group R of the alkoxy polyethylene glycol $RO(CH_2CH_2O)_nH$ contains from 1 to 20 carbon atoms, for example R may be methyl or butyl. A mixture of compounds having different R groups, for example a mixture of $C_7$ to $C_9$ alkyl groups may also be used. n is at least 5 and is preferably from 5 to 120, for example 5 to 25. It will be appreciated that n will normally represent

the average of a mixture of isomers of varying degrees of polymerisation and will not in general therefore be an integer.

An especially suitable ~~alkoxy~~ polyethylene glycol is methoxy polyethylene glycol (in which R is methyl), which preferably has a molecular weight in the range 300 to 1000.

Examples of organic di- or polyisocyanates which may be reacted with the methoxy polyethylene glycol and the isocyanate-reactive compound (if used) to form the surfactant include aliphatic isocyanates such as hexamethylene diisocyanate, aromatic isocyanates such as <u>m</u>- and <u>p</u>-phenylene diisocyanate, tolylene-2,4- and 2,6-diisocyanates, diphenylmethane-4,4'-diisocyanate, chlorophenylene-2,4-diisocyanate, naphthylene-1,5-diisocyanate, diphenylene-4,4'-diisocyanate, 4,4'-diisocyanate-3,3'-dimethyldiphenyl, 3-methyldiphenylmethane-4,4'-diisocyanate and diphenyl ether diisocyanate, cycloaliphatic diisocyanates such as cyclohexane-2,4- and 2,3-diisocyanates, 1-methyl cyclohexyl-2,4- and 2,6-diisocyanates and mixtures thereof and bis-(isocyanatocyclohexyl-)methane and tri-isocyanates such as 2,4,6-triisocyanatotoluene and 2,4,4'-triisocyanatodiphenylether.

Mixtures of isocyanates may be used, for example a mixture of tolylene diisocyanate isomers such as the commercially available mixtures of 2,4- and 2,6-isomers and also the mixture of di- and higher poly-isocyanates produced by phosgenation of aniline/formaldehyde condensates. Such mixtures are well known in the art and include the crude phosgenation products containing mixtures of methylene bridged polyphenyl polyisocyanates, including diisocyanate, triisocyanate and higher polyisocyanates together with any phosgenation byproducts.

Preferred polyisocyanates to be used in the present invention are those wherein the isocyanate is an aromatic diisocyanate or polyisocyanate of higher functionality in particular crude mixtures of methylene bridged polyphenyl polyisocyanates containing diisocyanates, triisocyanate and higher functionality polyisocyanates. Methylene bridged polyphenyl polyisocyanates are well known in the art and have the generic formula:

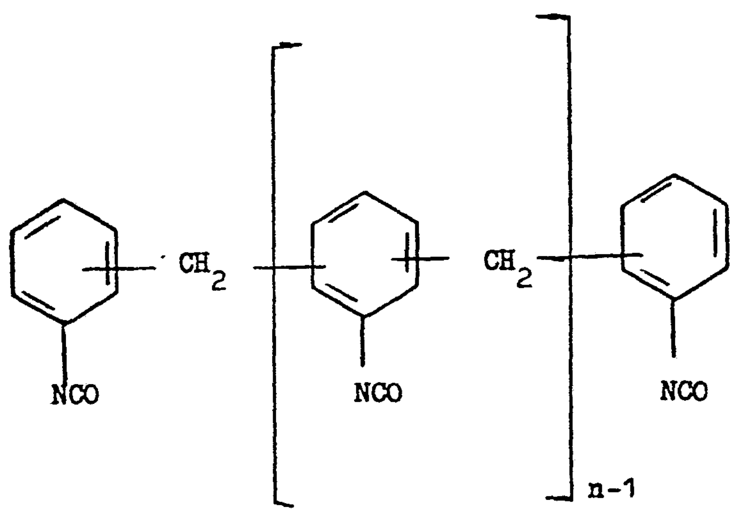

where n is one or more and in the case of the crude mixtures represents an average of more than one. They are prepared by phosgenation of corresponding mixtures of polyamines obtained by condensation of aniline and formaldehyde. For convenience, crude mixtures of methylene bridged polyphenyl polyisocyanates containing diisocyanate, triisocyanate and higher functionality polyisocyanates are referred to hereinafter as "crude MDI".

The isocyanate-reactive compound having one or more hydroxy, amino or carboxylic acid groups may for example be a monofunctional alcohol, (for example methanol, ethanol, benzyl alcohol or phenol) a monofunctional amine (for example aniline or benzylamine), or a monomeric glycol or polyol, diamine or polyamine, a hydroxyl-ended polyester or hydroxyl-ended polyether.

Examples of suitable monomeric glycols or polyols include: ethylene glycol, propylene glycol, butane diol, pentane diol, hexane diol, dipropylene glycol, tripropylene glycol, oxypropylated bisphenol A, oxyethylated bisphenol A, oxyethylated hydroquinone and glycerol.

Examples of suitable diamines or polyamines include: tolylene diamine and hexamethylene diamine.

The surfactant may be prepared by reacting the di- or polyisocyanate with the alkoxy polyethylene glycol (and the isocyanate-reactive compound if used), there being at least one molar proportion *in toto* of the alkoxy polyethylene glycol and the isocyanate-reactive group(s) for each isocyanate functional group of the di- or polyisocyanate, whereby the surfactant product has substantially no isocyanate functionality.

If an isocyanate-reactive compound is used in addition to the alkoxy polyethylene glycol, it is preferred that the reaction takes place in two stages, the alkoxy polyethylene glycol being reacted with the di- or polyisocyanate first, and that reaction product being subsequently reacted with the isocyanate-reactive compound. The reaction mixture is preferably heated at a temperature of 50° to 150°C.

The proportions of the isocyanate-reactive compound may be varied within wide limits if it is difunctional (e.g. a glycol) to give a corresponding range of surfactants of varying properties. Typical molar ratios of alkoxy polyethylene glycol : di- or polyisocyanate : isocyanate-reactive compound lie in the range 2:1.1:0.1 to 2:4:3.

The organic isocyanate which is emulsified in the presence of the surfactant to form the aqueous emulsion may be any suitable isocyanate selected for the desired application to which the emulsion is to be put. Suitable organic isocyanates include those listed above, for example MDI, crude MDI or polymers or oligomers thereof formed for example by reaction with a monomeric glycol or polyol (or mixtures thereof) or with a hydroxyl-ended polyester or polyether (MDI 'prepolymers'). Also included are "modified" isocyanates in which a proportion of the isocyanate groups are converted into other functional groups such as carbodiimide groups or uretonimine groups, or in which a proportion of the isocyanate groups are reacted with any of a wide range of possible isocyanate-reactive compounds.

The surfactant is preferably admixed with water. The organic isocyanate is then added to this solution to form the emulsion. However, the surfactant may alternatively be admixed with the organic isocyanate and stored until required, at which point water is added to form the emulsion.

According to a further aspect of the present invention there is thus provided an emulsifiable isocyanate composition comprising an admixture of an organic isocyanate and a surfactant which is the reaction product of an organic di- or polyisocyanate with:
(1) an alkoxy polyethylene glycol of formula $RO(CH_2CH_2O)_nH$ wherein R is an alkyl group of 1 to 20 carbon atoms, and n is at least 5, and, optionally,
(2) an isocyanate-reactive compound having one or more hydroxy, amino or carboxylic acid groups,
wherein the proportions of the said isocyanate-reactive compound (2) are from 0 to 50 parts by weight of isocyanate-reactive compound per 100 parts by weight of alkoxy polyethylene glycol (1), and wherein the isocyanate function of the organic di- or polyisocyanate is completely reacted with the alkoxy polyethylene glycol and the isocyanate-reactive compound (if used).

The emulsions according to the invention are of the oil in water type and preferably comprise from 99 parts to 25 parts by weight of water, from 1 part to 75 parts by weight of organic polyisocyanate and a stabilising amount of surface active agent hereinbefore described. A preferred amount of the surface active agent is from 1% by weight to 10% by weight based on the organic isocyanate present.

Emulsions comprising approximately equal parts by weight of water and organic polyisocyanate are useful for most applications.

The invention further provides a process for preparing an aqueous emulsion comprising the steps of mixing an organic isocyanate with water in the presence of a surface active agent as hereinbefore described. Mixing of the organic isocyanate with the water may be carried out by any means which is appropriate for emulsification.

Optionally, emulsions according to the invention may comprise an emulsifying agent additional to the surfactants described hereinbefore. Such additional emulsifying agents or stabilisers retard the rate of settling of the emulsion and assist in the prevention of any tendency for the emulsions to flocculate. Preferred stabilisers are polyethylene oxide, hydroxypropyl guar gum, cellulose gums and polypeptides such as gelatin or bone glue. Stabilisers may preferably be present at a concentration of 0.01% to 1.0% by weight based on the water present. Conveniently, the stabilisers are incorporated into the emulsions by dissolving or dispersing them in water, which may be the whole, or part, of the water used in preparing the emulsion.

When the surfactant is admixed with water prior to emulsification of the organic isocyanate, dilute compositions, for example those containing 99 parts to 90 parts by weight of water and 1 part to 10 parts by weight of surface active agant and (when present) of stabiliser may conveniently be used as they are for emulsifying the organic isocyanate. More concentrated compositions, for example those containing from 40 parts to 70 parts by weight of water and 60 parts

to 30 parts by weight of surface active agent and (when present) of stabiliser may be diluted appropriately with water (for example with a dilution factor of up to 20 to 25 times) and thereafter used to emulsify the organic isocyanate.

It may be desirable to incorporate a solubilising compound, for example urea, in the more concentrated compositions.

The emulsions of the present invention are useful as adhesives, binders and surface coatings. Examples of suitable applications are given in UK Patent Specification Nos.1444933 and 1502777.

In our pending UK Patent Application No.10264/75 published as Belgian Patent No.839546 there is described and claimed a process for manufacturing sheets or moulded bodies, especially chipboard, which comprises hot pressing a mass of lignocellulosic material mixed with a binding agent comprising an aqueous emulsion of an organic polyisocyanate. The emulsions of the present invention are particularly useful for this application where delays of a few hours in pressing can occur.

Thus according to a further aspect of our invention we provide a process for manufacturing sheets or moulded bodies (for example chipboard) comprising hot pressing a mass of lignocellulosic material mixed with an aqueous emulsion of the invention.

Lignocellulosic material which can be used in the process includes wood chips, wood fibres, shavings, wood wool, cork and bark, sawdust and like waste products from the woodworking industry, and/or fibres from other

natural products which are lignocellulosic for example bagasse, straw, flax residues and dried rushes, reeds and grasses. Additionally, there may be mixed with the lignocellulosic material inorganic flake or fibrous material e.g.glass fibre, mica or asbestos.

The process is conveniently carried out by spraying the lignocellulosic material with the emulsion while it is being tumbled in a mixer. The sprayed chips are allowed to fall evenly onto a steel platen coated with a release agent and pressed at a temperature of from 150 to 220°C. The isocyanate emulsion may be used in conjunction with other binders such as the resin glues commonly used for chipboard manufacture. It may also be used in admixture with, for example, urea-formaldehyde resin glue. A useful mixture comprises 2 to 3 parts urea-formaldehyde and 1 part of emulsifiable isocyanate. Additives and adjuvants commonly used in isocyanate compositions may be incorporated in the emulsions as desired. Pigments, fillers, antioxidants, resins, and plasticisers are examples. Hydrophobic diluents may be useful in further slowing down the rate of reaction.

The invention is illustrated by the following Examples.

Examples 1 - 7

420 parts of methoxy polyethylene glycol of molecular weight 750 (MOPEG 750) was added with stirring to 70 parts of 4,4'-diphenylmethane diisocyanate at 70°C. Stirring at 70°C was maintained for 2 hours.

The above procedure was repeated with the following isocyanates as shown in Table 1:-

| Experiment | | Isocyanate |
|---|---|---|
| 1 | A | 4,4'-diphenylmethane diisocyanate |
| 2 | B | 4,4'-diphenylmethane diisocyanate modified by reaction with a mixture of low M.W. diols to an isocyanate value of 23.1%. |
| 3 | C | 4,4'-diphenylmethane diisocyanate in which there has been partial conversion of the isocyanate groups to carbodiimide groups to give a product of isocyanate value 29.5%. |
| 4 | D | A blend of approximately equal weights of a crude MDI and a prepolymer prepared from diphenylmethane diisocyanate and mixture of low M.W. diols, isocyanate value of the blend 28.5%. |
| 5 | E | A blend in the approximate weight ratio 3:1 of a crude MDI and a prepolymer prepared from diphenylmethane diisocyanate and a mixture of a low M.W. diol and a low M.W. polyether, isocyanate value of the blend 29.0%. |
| 6 | F | A crude MDI of viscosity 140-200 centipoises containing 48% diphenylmethane diisocyanates. |
| 7 | G | A crude MDI of viscosity 800 to 1000 centipoises containing 33% diphenylmethane diisocyanates. |

Emulsions were prepared from 2.4 parts of the surfactants of Experiments 1 to 7, 60 parts of water and 60 parts of isocyanate F above. In a first set of experiments, the surfactant was dissolved in the isocyanate phase, and in a second set of experiments the surfactant was dissolved in the water phase. A "Silverson" high speed stirrer was used to form the emulsions.

The "pot life" of these emulsions (the time taken for the emulsions to form a high viscosity paste) is shown in Table 1.

Methoxy polyethylene glycols are hereinafter referred to as MOPEG followed by the average number molecular weight.

Table 1

| Example | Isocyanate | Wt. Isocyanate (parts) | Wt. MOPEG 750 (parts) | POT LIFE (Hours, mins.) | |
|---|---|---|---|---|---|
| | | | | Surfactant in Isocyanate | Surfactant in water |
| 1 | A | 70 | 420 | 4.00 | 6.30 |
| 2 | B | 102.5 | 420 | 4.00 | 6.00 |
| 3 | C | 81.5 | 420 | 3.30 | 5.15 |
| 4 | D | 83.5 | 420 | 3.45 | 5.30 |
| 5 | E | 82.3 | 420 | 3.50 | 5.30 |
| 6 | F | 77.0 | 420 | 4.00 | 5.30 |
| 7 | G | 77.0 | 420 | 4.00 | 5.30 |

Examples 8 - 15

363 parts of MOPEG 750 was added with stirring to 132 parts of isocyanate F of Example 6 maintained at 70°C. After the initial exotherm, 15 parts of ethylene glycol was added, and stirring at 70°C was maintained for 2 hours.

The above procedure was repeated with the glycols shown in Table 2 to form the Surfactants of Experiments 8 to 15.

Emulsions were prepared as in Examples 1 - 7 above, and the pot life of the emulsions is given in Table 2.

Table 2

| Ex. | Glycol | Weight of glycol (parts) | Weight of isocyanate F (parts) | Weight of MOPEG 750 (parts) | POT LIFE (hours. minutes) Surfactant in isocyanate | Surfactant in water |
|---|---|---|---|---|---|---|
| 8 | ethylene | 15 | 132 | 363 | 6.00 | 5.00 |
| 9 | 1,2-propylene | 18.3 | 132 | 363 | 6.30 | 6.00 |
| 10 | 1,4-butane | 21.5 | 132 | 363 | 5.45 | 6.00 |
| 11 | 1,5-pentane | 24.7 | 132 | 363 | 5.00 | 5.30 |
| 12 | 1,6-hexane | 27.9 | 132 | 363 | 5.00 | 5.00 |
| 13 | Di-propylene | 31.4 | 132 | 363 | 4.45 | 4.45 |
| 14 | Tri-propylene | 43.8 | 132 | 363 | 4.30 | 5.00 |
| 15 | oxy-propylated bisphenol A | 73.4 | 132 | 363 | 6.00 | 5.30 |

Examples 16 to 18

150 parts of MOPEG 750 was added with stirring to 27.5 parts of isocyanate F of Example 6 maintained at 70°C. Stirring at 70°C was maintained for 2 hours. This procedure was repeated with methoxy polyethylene glycols (MOPEG) of different molecular weights, and emulsions were prepared from the resultant surfactants as in Examples 1 - 6. The "pot lives" of the emulsions formed are given in Table 3.

Table 3

| Example | Methoxy Polyethylene Glycol (MOPEG) | Weight MOPEG (parts) | Weight Isocyanate F (parts) | POT LIFE (Hours, minutes) | |
|---|---|---|---|---|---|
| | | | | Surfactant in isocyanate | Surfactant in water |
| 16 | MOPEG 750 | 150 | 27.4 | 4.00 | 5.30 |
| 17 | MOPEG 550 | 110 | 27.4 | 4.45 | 4.45 |
| 18 | MOPEG 350 | 70 | 27.4 | 4.30 | 6.00 |

Examples 19 to 21

The procedure of Examples 8 to 15 was repeated using MOPEG of molecular weight 550 and diethylene glycol in the proportions shown in Table 4. The pot lives of the resultant emulsions are also shown in Table 4.

Table 4

| Example | MOPEG 550 (parts) | Diethylene glycol (parts) | Isocyanate F (parts) | POT LIFE (Hours, minutes) | |
|---|---|---|---|---|---|
| | | | | Surfactant in Isocyanate | Surfactant in Water |
| 19 | 110 | - | 27.4 | 4.45 | 4.45 |
| 20 | 110 | 10.6 | 54.8 | 4.45 | 4.30 |
| 21 | 110 | 21.2 | 72.2 | 4.45 | 4.30 |

Examples 22 and 23

The procedure of Examples 8 to 15 was repeated replacing the glycol with benzyl alcohol and aniline respectively. The proportions and the "pot lives" of the resultant emulsions are given in Table 5.

Table 5

| Ex. | Isocyanate reactive compound | Weight isocyanate reactive compound (parts) | Weight isocyanate F (parts) | Weight MOPEG 750 (pts.) | POT LIFE (hours, minutes) Surfactant in Isocyanate | Surfactant in water |
|---|---|---|---|---|---|---|
| 22 | benzyl alcohol | 21.6 | 27.4 | 75 | 4.00 | 5.45 |
| 23 | aniline | 9.3 | 27.4 | 75 | 5.30 | 5.45 |

Examples 24 - 27

2 parts of the surfactant of Example 6 was dissolved in 100 parts of water containing 0.25 parts of the stabilisers shown in Table 6.

The pot life and the time for the emulsions to settle to a predetermined level were as given in Table 6.

Table 6

| Example | Stabiliser | Pot life | Settling time |
|---|---|---|---|
| 24 | Cellulose gum (Hercules 7M275) | >5 hours | non settling |
| 25 | Polyethylene oxide (Polyox WSR 205) | >5 hours | non settling |
| 26 | Gelatin | >5 hours | 44 minutes |
| 27 | Bone glue | >5 hours | 50 minutes |

Example 28

1.5 parts of polyethylene oxide (Polyox WSR 205) was dissolved in 68.5 parts of water, and the surfactant of Example 6 (30 parts) was added to give a clear solution.

6.7 parts of the concentrate prepared above were added to 95.5 parts of water. 100 parts of isocyanate F

of Example 6 were added and an emulsion was formed using a high speed Silverson stirrer.

The loss of isocyanate strength of the emulsion with time was determined by reaction with di-n-butylamine and back titration with hydrochloric acid at periods of 1 hour, 2 hours, 4 hours and 6 hours after the formation of the emulsion. The results are shown in Table 7.

Example 29

6.7 parts of the concentrate prepared as in Example 28 above was added to 79 parts of water. 100 parts of isocyanate F of Example 6 were added and emulsified using a Silverson stirrer. 33 parts of a commercially available 50% wax emulsion (Mobil ED 80/196) was added using a low speed stirrer.

The loss of isocyanate strength of the mixed emulsion was determined as in Example 28, and the results are shown in Table 7.

Table 7

| Example | ISOCYANATE STRENGTH AFTER HOURS: | | | | |
|---|---|---|---|---|---|
| | 0 | 1 | 2 | 4 | 6 |
| 28 | 30.9 (100%) | 30.5 (99%) | 30.2 (98%) | 30.0 (97%) | 28.6 (93%) |
| 29 | 30.9 (100%) | 30.7 (99%) | 29.8 (96%) | 29.1 (94%) | 27.2 (88%) |

Example 30

A surfactant-stabiliser concentrate was prepared from 2 parts of polyethylene oxide (Polyox WSR 205), 58 parts of water and 40 parts of the surfactant of Example 6. After leaving overnight, this concentrate separated into two layers.

The above preparation was repeated but with the further addition of 5 parts urea. The urea was dissolved in the water first, prior to the addition of the polyox and surfactant.

No separation into layers was apparent on storage.

Example 31

6.7 parts of the concentrate of Example 28 was added to 95.5 parts of water.

100 parts of the following isocyanates were emulsified in solutions prepared as above:

Isocyanate C of Example 3

Isocyanate D of Example 4

Isocyanate E of Example 5

Isocyanate G of Example 7

All gave stable emulsions with "pot lives" in excess of 5 hours.

Example 32

(a) A wood chip panel was prepared using an isocyanate emulsion as binder.

The formulation used was:

| | |
|---|---|
| Pine wood chip (containing 2% moisture) | 1020 parts |
| water | 60 parts |
| Isocyanate F | 60 parts |
| Surfactant concentrate as in Example 28 | 2 parts |

The surfactant concentrate described in Example 28 was diluted with the 60 parts of water and an emulsion was formed with the isocyanate.

This emulsion was sprayed onto the wood chip using a conventional blender. The glued wood chips were spread in a former measuring 40 cm x 30 cm. placed on top of a steel plate.

The steel plate was coated with a solution of soft soap as a release agent. The former was gently removed and a similar coated steel plate placed on top of the wood chip cake. The cake was then placed in a press at 175°C with 19 mm spacers. The cake was pressed for 3 minutes 48 seconds.

The resulting panel had the properties shown in Table 8.

(b) A similar board was prepared but the isocyanate emulsion was left standing for 4 hours before spraying onto the wood chips.

The resulting panel had the properties shown in Table 8.

Table 8

| Example | 8a | 8b |
|---|---|---|
| Density ($Kgm^{-3}$) | 685 | 690 |
| 2 hr. water swelling (%) | 16 | 16 |
| 24 hr. water swelling (%) | 19 | 19 |
| Perpendicular tensile strength (V20)($kNm^{-2}$) (DIN 68763) | 1000 | 1050 |
| Perpendicular tensile strength (V100)($kNm^{-2}$) (DIN 68763) | 300 | 370 |

## CLAIMS

1. An aqueous emulsion comprising an organic isocyanate and a surfactant characterised in that the surfactant is the reaction product of an organic di- or polyisocyanate with
(1) an alkoxy polyethylene glycol of formula $RO(CH_2CH_2O)_nH$ wherein R is an alkyl group of 1 to 20 carbon atoms, and n is at least 5 and, optionally,
(2) an isocyanate-reactive compound having one or more hydroxy, amino or carboxylic acid groups,
wherein the proportions of the said isocyanate-reactive compound (2) are from 0 to 50 parts by weight of isocyanate-reactive compound per 100 parts by weight of alkoxy polyethylene glycol (1), and wherein the isocyanate function of the organic di- or polyisocyanate is completely reacted with the alkoxy polyethylene glycol and the isocyanate-reactive compound (if used).

2. An aqueous emulsion according to claim 1 wherein the alkoxy polyethylene glycol is a methoxy polyethylene glycol having a molecular weight in the range 300 to 1000.

3. An aqueous emulsion according to claim 1 or claim 2 wherein the organic polyisocyanate used to make the surfactant is a crude mixture of methylene bridged polyphenyl polyisocyanates.

4. An aqueous emulsion according to any one of the preceding claims wherein the organic isocyanate present therein comprises diphenylmethane diisocyanate.

5. An aqueous emulsion according to any one of the preceding claims comprising from 99 to 25 parts by weight of water, from 1 to 75 parts by weight of the organic isocyanate and a stabilising amount of the surfactant.

6. An aqueous emulsion according to claim 5 wherein the amount of surfactant is from 1 to 10% by weight based on the weight of organic isocyanate.

7. A process for manufacturing sheets or moulded bodies which comprises hot pressing a mass of lignocellulosic material mixed with an aqueous emulsion according to any one of the preceding claims.

8. An emulsifiable isocyanate composition comprising a mixture of an organic isocyanate and a surfactant characterised in that the surfactant is the reaction product of an organic di- or polyisocyanate with:
(1) an alkoxy polyethylene glycol of formula $RO(CH_2CH_2O)_nH$ wherein R is an alkyl group of 1 to 20 carbon atoms, and n is at least 5, and, optionally,
(2) an isocyanate-reactive compound having one or more hydroxy, amino or carboxylic acid groups,
wherein the proportions of the said isocyanate-reactive compound (2) are from 0 to 50 parts by weight of isocyanate-reactive compound per 100 parts by weight of alkoxy polyethylene glycol (1), and wherein the isocyanate function of the organic di- or polyisocyanate is completely reacted with the alkoxy polyethylene glycol and the isocyanate-reactive compound (if used).

MJR/BH
12.12.79.

European Patent Office

# EUROPEAN SEARCH REPORT

0013112
Application number
EP 79 30 2920

## DOCUMENTS CONSIDERED TO BE RELEVANT

| Category | Citation of document with indication, where appropriate, of relevant passages | Relevant to claim |
|---|---|---|
| D,A | GB - A - 1 444 933 (I.C.I.)<br>* Page 4, claims 1,4,7,8; page 3, lines 10-13 *<br>-- | 1 |
| A | FR - A - 2 303 661 (I.C.I.)<br>* Page 14, claims 1-7 * | 1 |
| D | & BE - A - 839 546<br>-- | |
| A | FR - A - 1 157 828 (CIBA)<br>* Page 4, summary points I and $I_2$; page 1, right-hand column, paragraph 2 *<br>---- | 1 |

CLASSIFICATION OF THE APPLICATION (Int. Cl.):
C 07 C 119/042
C 08 G 18/70
C 08 L 97/02

TECHNICAL FIELDS SEARCHED (Int.Cl.):
C 07 C 119/042
C 08 G 18/70
18/30
18/10
18/12
18/28
C 08 L 97/02

CATEGORY OF CITED DOCUMENTS
X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 21-03-1980 | V. PUYMBROECK |

EPO Form 1503.1 06.78